# EUROPEAN PATENT APPLICATION

(11) **EP 4 529 843 A1**
(43) Date of publication of application: **02.04.2025**
(21) Application number: 23199615.8
(22) Date of filing: 26.09.2023
(51) Int. Cl.: A61B 6/00

(54) **METHOD FOR ACQUIRING IMAGES BY A MEDICAL IMAGING SYSTEM**

(71) Applicant: Koninklijke Philips N.V., 5656 AG Eindhoven (NL)
(72) Inventor: BYSTROV, Daniel, 5656AG Eindhoven (NL); KROENKE-HILLE, Sven, 5656AG Eindhoven (NL); YOUNG, Stewart Matthew, 5656AG Eindhoven (NL); VON BERG, Jens, 5656AG Eindhoven (NL); BRUECK, Heiner Matthias, 5656AG Eindhoven (NL); GOOßEN, Andre, 5656AG Eindhoven (NL); HARDER, Tim Philipp, 5656AG Eindhoven (NL); SENEGAS, Julien Thomas, 5656AG Eindhoven (NL)
(74) Representative: Philips Intellectual Property & Standards

(57) **Abstract**

The invention relates to a medical imaging system (100) comprising: an imaging device (1) comprising at least one radiation source (7) configured to emit radiation (11) towards a target of interest (50) and at least one radiation detector (9) configured to detect the emitted radiation (11), wherein the medical imaging system (100) is configured to acquire at least one first two-dimensional image of the target of interest (50) at an at least one first source image distance, SID (3), and at least one second two-dimensional image of the target of interest (50) at an at least one second SID (5) different to the first SID (3), and a reconstruction means (150) configured to reconstruct at least partially at least one three-dimensional image based on the first and second two-dimensional images.

## Description

### FIELD OF THE INVENTION

The present invention relates to a method for acquiring images by a medical imaging system, a medical imaging system, a computer program product, and a computer readable medium.

### BACKGROUND OF THE INVENTION

In the context of medical imaging systems, such as x-ray imaging systems, while x-ray imaging is often the entry point to the diagnostic pathway for a patient, follow-up three-dimensional (3D) exams like exams based on computer tomography (CT) and magnetic resonance (MR) must be frequently ordered to resolve ambiguities in the interpretation of findings in the two-dimensional (2D) x-ray image. Such exams are often expansive, costly and come with additional dose (e.g. in CT) for the patient.

Furthermore, conventional x-ray imaging results in a purely 2D projective image which may feature ambiguities that can only be resolved in 3D.

3D imaging modalities may resolve such ambiguities but are generally costly, technically extraordinarily complex and come with additional patient dose, e.g. in the case of CT. Thus, if possible, such 3D follow-up exams should be avoided.

Moreover, the quality of the x-ray image highly depends on the positioning of the patient relative to the detector and x-ray source. In particular in conventional tomosynthesis, the relative position between patient and source is often needed to be tracked externally, e.g., by optical fibers.

To resolve spatial ambiguities, different views on the same anatomy are acquired conventionally (e.g., chest posterior-anterior and lateral acquisitions) or conventional tomosynthesis is applied, which both often leads to a higher radiation dose and are rather complex in the application.

Thus, it is an object of the invention to provide an improved medical imaging method and system, which addresses the above noted drawbacks of the prior art.

### SUMMARY OF THE INVENTION

The invention provides a method for acquiring images by a medical imaging system, a medical imaging system, a computer program product, and a computer readable medium according to the independent claims. Preferred embodiments are laid down in the dependent claims.

The present invention relates to a method for acquiring images by a medical imaging system.

The method comprises the step of acquiring, by at least one imaging device, at least one first two-dimensional image of a target of interest at an at least one first source image distance, SID.

The method further comprises the step of acquiring, by the imaging device, at least one second two-dimensional image of the target of interest at an at least one second SID different to the first SID.

The method further comprises the step of reconstructing, at least partially, by a reconstruction means, at least one three-dimensional image based on the first and second two-dimensional images.

In other words, in the method according to the present invention may allow to obtain a three-dimensional image by the reconstruction of at least two two-dimensional images acquired at two different SIDs.

Contrary to prior art methods and devices, the method according to the present invention may allow to obtain 3D information by slightly varying the SID and acquiring at least two potentially low-dose x-ray images with distinct SIDs. A radiation dose for each acquisition may be accordingly much lower compared to conventional imaging techniques, for instance compared to a full conventional x-ray exam.

Unlike prior art proposals, the relative position between patient and source may not need to be tracked externally, since the position information may already be known or provided by standard parameters of the medical imaging system, such as the dimensions and arrangements of the parts of the medical imaging system.

An SID may be a distance between a radiation emitting source and a corresponding radiation detector, for instance a distance orthogonal to a main surface of the radiation detector, wherein the main surface may be the surface upon which the target of interest is arranged. However, an SID may also be a distance between a radiation emitting source and a corresponding radiation detector, for instance a distance at an oblique angle to the main surface of the radiation detector.

According to the present invention, one or more image devices may be employed to acquire the images. Thus, the acquisition may be performed by a single device, which may accordingly adapted to allow an acquisition of one or more images with at least two different SIDs. This may be, for instance, achieved by moving the imaging device from a first position defining a first SID at which a first image is acquired to a second position defining a second SID at which a second image is acquired, wherein the second SID is different to the first SID. Thus, the first and second image acquisition may be performed by the same device.

However, also more than one image device may be provided in the medical imaging system according to the present invention for allowing an acquisition of one or more images at least two different SIDs. This may be, for instance, achieved by the arranging the first imaging device at a first position defining a first SID at which a first image is acquired and by the arranging the second imaging device a second position defining a second SID at which a second image is acquired, wherein the second SID is different to the first SID.

The method according to the present invention may comprise the acquisition of a first two-dimensional image of a target of interest and the acquisition of second two-dimensional image of the target of interest. The reconstructing step of the three-dimensional image may then be based on the first and second two-dimensional images.

However, also more than one first and/or more than one second dimensional images of the target of interest may be acquired. The reconstruction may then be based also on the additional two-dimensional images.

The two-dimensional images may be projective images and/or may be absorption images of the target of interest.

A medical imaging system according to the present invention, may be any system that may allow for an examination of a medical target of interest, which may be for instance a part of a body or a full body of a patient.

The first acquisition step and the second acquisition step may be performed sequentially, i.e. at different times, or simultaneously, i.e. at the same time.

The reconstruction step may be performed such that a full reconstruction of a three-dimensional image of the target of interest may be obtained. Or, the reconstruction step may be performed partially. Thus, a partial reconstruction of a three-dimensional image of the target of interest may be obtained.

The reconstruction step may also be configured such that more than one three-dimensional images may be obtained.

Furthermore, the method according to the present invention may also include further acquisition steps and/or further reconstructing steps.

A reconstruction means may be any suitable means configured to reconstruct, based on the image data obtained by the preceding image acquisition steps, a three-dimensional image. This may be achieved by suitable AI or machine learning means comprising one or more suitable algorithms to proceed the acquired image data and reconstruct a three-dimensional image based on said image data accordingly.

The reconstruction may be based on a priori knowledge, e.g., in form of training data for an AI-based reconstruction algorithm.

Furthermore, in order to boost the signal-to-noise ratio, AI-based denoising techniques may be applied.

The method may be at least partly computer-implemented, and may be implemented in software or in hardware, or in software and hardware.

Further, the method may be carried out by computer program instructions running on means that provide data processing functions. The data processing means may be a suitable computing means, such as an electronic control module etc., which may also be a distributed computer system. The data processing means or the computer, respectively, may comprise one or more of a processor, a memory, a data interface, or the like.

The method and the medical imaging system according to the present invention may particularly overcome drawbacks of conventional imaging techniques such as tomosynthesis by that, for instance, only slightly different views may be acquired.

In contrast to conventional imaging systems and methods, the method and the medical imaging system according to the present invention may allow for a seamless integration into a conventional x-ray exam workflow, without the patient even noticing any difference during the exam, and can also be optionally disabled.

In a preferred embodiment of the method of present invention, a difference from the first SID and the second SID is in a range of 50 mm to 500 mm.

An SID may be, for instance, provided in the range of 1000 mm to 3000 mm. Given the preferred range of an SID variation of 50 mm to 500 mm, a final depth resolution of approximately 1 mm may be accordingly obtained considering a focal spot size of approximately 0.6 mm to 1.2 mm.

In a preferred embodiment of the method of the present invention, a single first two-dimensional image at the first SID and a single second two-dimensional image at the second SID is acquired, and the three-dimensional image is reconstructed based on the single first and second two-dimensional images.

In other words, only one single image may be acquired for each SID. Thus, no further two-dimensional images are acquired and used for the reconstruction of the three-dimensional image. This may allow for a particular low dose imaging since no further two-dimensional images are acquired.

In a preferred embodiment of the method of the present invention, the first two-dimensional image is acquired at a first position of a radiation source, and the second two-dimensional image is acquired at a second position of the radiation source, wherein a tomographic angle defined between the first position and the second position is essentially zero.

The radiation source may emit the radiation at the time of acquisition of the first two-dimensional image towards the target of interest in the first position along a first radiation axis. Further, the radiation source may emit the radiation at the time of acquisition of the second two-dimensional image towards the target of interest in the second position along a second radiation axis. The tomographic angle may be understood as an amplitude of traveling of the radiation source between the first and second radiation axes measured in degrees.

The method according to the present invention may accordingly allow to acquire the first and second two-dimensional images at a same lateral position but at different SIDs, wherein the tomographic angle defined between the first position and the second position is essentially zero. Thus, potential errors and ambiguities, which may be introduced by a potential misplacement of the imaging device at unwanted angles, may be avoided. Since the tomographic angle is essentially zero, only the SIDs may have to be varied to acquire the first and second two-dimensional images for finally obtaining a reconstructed three-dimensional image of the target of interest.

This may also allow to simplify the imaging system because any means for an angle adaption such as, for instance, motors and corresponding control means, which are usually necessary to set different tomographic angles, may be omitted.

Nevertheless, it may be also possible to acquire first and second two-dimensional images at a tomographic angle that is essentially zero and to further acquire further two-dimensional images with tomographic angles that are greater than zero.

In a preferred embodiment of the method of the present invention, the first two-dimensional image is acquired at a first position of a radiation source, and the second two-dimensional image is acquired at a second position of the radiation source, wherein a tomographic angle defined between the first position and the second position is greater than zero.

Setting a tomographic angle greater than zero during image acquisition in addition to different SIDs may allow for an improved reconstruction of the three-dimensional image. Compared to prior art approaches, the imaging method according to the present invention may allow that only relative small tomographic angles could be sufficient for obtaining a sophisticate reconstructed three-dimensional image.

Further, when a radiation beam, preferably a central radiation beam, is varied with the acquisitions at different SIDs, introducing a small tomographic angle, the ability of the method and system according to the present invention to spatially resolve certain structures along the beam may be improved.

In a preferred embodiment of the method of the present invention, the first two-dimensional image is acquired at a first acquisition time, the second two-dimensional image is acquired at a second acquisition time, and the three-dimensional image is reconstructed based on the images and acquisition times of the first and second two-dimensional images.

Thus, the first acquisition time may be the time at which the first two-dimensional image is acquired. This may include a distinct time or duration at which a radiation is emitted and accordingly detected.

Likewise, the second acquisition time may be the time at which the second two-dimensional image is acquired. This may include a distinct time or duration at which a radiation is emitted and accordingly detected. The first and second acquisition times may be the same time and duration or different times and durations.

Also further images may be acquired at further times or durations. Furthermore, a time-sequence of at least two images *Xᵢ* (with i indexing the acquisition time), which may be for instance x-ray images, may be acquired.

The present invention also relates to a medical imaging system.

The medical imaging system comprises an imaging device comprising at least one radiation source configured to emit radiation towards a target of interest and at least one radiation detector configured to detect the emitted radiation.

The medical imaging system is configured to acquire at least one first two-dimensional image of the target of interest at an at least one first SID.

The medical imaging system is further configured to acquire at least one second two-dimensional image of the target of interest at an at least one second SID different to the first SID.

The medical imaging system further comprises a reconstruction means configured to reconstruct at least partially at least one three-dimensional image based on the first and second two-dimensional images.

In other words, in the claimed medical imaging system may allow to obtain a three-dimensional image by the reconstruction of at least two two-dimensional images acquired with at least two different SIDs.

A medical imaging system may be any system that may allow to image medical targets of interest, such as, for instance portions of a body or a body, e.g. of a patient.

An imaging device may be a device or an arrangement of a plurality of devices that may allow for a multi-dimensional imaging of a target of interest.

The reconstruction means may be any device or software allowing for a respective reconstruction of a three-dimensional image based on the first and second two-dimensional images. This may include, but is not limited to, a computer comprising a respective software for reconstruction.

The radiation source may be any suitable source for emitting electromagnetic radiation suitable to perform medical imaging, for instance an x-ray source. Also more than one radiation source may be provided.

The effective variation of the SID may be preferably fast enough so that no patient-motion artifacts are to be effective. Likewise the effective variation of the SID may be preferably slow enough that absorption images can be recorded by the given detector.

The radiation detector may be any suitable detector for receiving and detecting electromagnetic radiation for performing medical imaging, for instance an x-ray detector. Also more than one radiation detector may be provided. The radiation detector may comprise fast enough read-out times and may be, for instance, a fluoroscopy detector or a photon-counting detector. Such quick detectors may allow to continuously detect while the SID is varied (i.e., a quasi-continuum of absorption images).

The components of the medical imaging device, such as, for instance, the radiation source or the radiation detector, may be configured to be moved along predetermined paths and/or along paths that may be tracked. Moreover, the components of the medical imaging device, such as, for instance, the radiation source or the radiation detector, may be moved prior to imaging, for example for adjusting the settings of the imaging system to the target of interest to be acquired and/or to set the distinct Field of View, FoV.

Further components may be provided at the medical imaging device such as one or more shutters and/or a support structure. The support structure may be a structure to which the radiation source and/or the radiation detector and/or further components of the medical imaging system are mounted and/or in which the radiation source, the radiation detector and/or further components are incorporated, such as a tube head or a C-arm.

In a preferred embodiment of the medical imaging system, the imaging device is an x-ray imaging device comprising the radiation source, which is an x-ray radiation source, wherein the x-ray radiation source is configured to generate and emit x-ray radiation.

Also more than one x-ray radiation source may be provided. The x-ray radiation source may be configured to emit an x-ray radiation beam of the defined shape towards the target of interest such that a distinct area of the target of interest could be irradiated. The x-ray radiation may be emitted for instance in form of a cone. The system according to the present invention may accordingly allow to acquire x-ray images at a very low dose.

In a preferred embodiment of the medical imaging system, the x-ray imaging device comprises an x-ray tube head, wherein the x-ray radiation source is arranged within the x-ray tube head, and wherein a variation between the first SID and the second SID is achieved by moving the x-ray tube head.

In a preferred embodiment, the variation between the first SID and the second SID is additionally or alternatively achieved by moving the x-ray radiation source within x-ray tube head.

Thus, at the first SID, which is defined by moving the x-ray tube head to the first position, at least one first two-dimensional image may be acquired. Further, at the second SID, which is defined by moving the x-ray tube head to the second position, at least one second two-dimensional image may be acquired.

Between the acquisitions of the first two-dimensional image and the second two-dimensional image, the x-ray tube head may also be shifted in a lateral direction, which may be essentially parallel or oblique to the surface of the x-ray radiation detector. The shifting may be performed by shifting the x-ray tube head by a fixed amount, preferably automatically, for instance, towards or away from the x-ray radiation detector.

Furthermore, an additional or alternative movement of the x-ray radiation source within x-ray tube head may allow for a more flexible positioning of the x-ray radiation source with respect to the target of interest and/or the radiation detector.

The x-ray imaging device may be optionally configured for CT imaging and/or for fluoroscopy. The x-ray imaging device may be a C-arm device.

In a preferred embodiment of the medical imaging system, a variation between the first SID and the second SID is additionally or alternatively achieved by varying an emitted x-ray cone such that different SIDs are simulated.

Thus, a change of the SID may be achieved by suitably varying and/or shaping the radiation cone, such as, for instance, by varying the divergence of the emitted x-ray cone. This may be achieved by a distinctive design of the x-ray tube and/or forming an electron beam.

This may allow for an acquisition of the first and second two-dimensional images without actually moving any part of the medical imaging system. Thus, the medical imaging system may not only be simplified further, but also potential errors and/or ambiguities may be avoided that may occur by misplacing the imaging device at distances to the detector and/or the target of interest.

In a preferred embodiment of the medical imaging system, the reconstruction means comprises at least one reconstruction algorithm adapted to reconstruct at least partially the at least one three-dimensional image based on the two-dimensional images,
wherein the reconstruction algorithm is based on one of the following:
training a deep-learning algorithm with computer tomography image data;
generalizing methods for three-dimensional rendering of discrete views to the domain of x-ray absorption images;
structure-from-motion techniques.

Accordingly, a three-dimensional image (or volume) may reconstructed.

Further a time-index i, which may be an index based on the acquisition time of the first and second two-dimensional images, may be associated with a corresponding SID of the respective first and second two-dimensional images, and may be accordingly denoted as SID*ᵢ*.

Furthermore, for the reconstruction of the three-dimensional image, also system-geometry parameters may be taken into account, which may be, for instance, known or measurable. The system-geometry parameters may comprise distances and arrangements between respective parts of the medical imaging system, for instance a distance between the radiation source and the radiation detector.

Accordingly, the acquisition geometry may be fully known for each measurement i. This information may then be fed into the reconstruction algorithm to map the {X,} to an at least partial 3D reconstruction of the imaged anatomy. This may allow to resolve structures along x-ray beam directions, which may be difficult or not resolvable in conventional x-ray images.

The reconstruction according to the present invention may help to resolve ambiguities occurring in a conventional x-ray image and might make it superfluous to acquire another view on the same anatomy (e.g., a lateral view in addition to the already acquired posterior-anterior view).

Furthermore, the same or a different algorithm may be provided to allow, based on the above noted data, for retrospectively changing the imaging geometry.

In a preferred embodiment of the medical imaging system, the reconstruction algorithm is additionally or alternatively designed to simulate an x-ray image with infinite SID based on the image data and the SID data.

Thus, an algorithm may be designed (or trained in the case of machine learning) to simulate an x-ray image with infinite SID (parallel projection) from the {(*SIDᵢ, Xᵢ*)} data. This may be particularly useful for avoiding perspective distortions in musculo-skeletal images which may impact the appearance of the joint gap.

Furthermore, it may allow for designing spatially more compact x-ray systems for thorax wall-stand exams which may require large SIDs.

In a preferred embodiment of the medical imaging system, no additional means are provided for tracking a distance between the target of interest and the radiation source and/or a position of the target of interest and the radiation source.

Thus, further means for tracking a distance between the target of interest and the radiation source and/or a position of the target of interest and the radiation source may be omitted. This may allow for a simplified medical imaging system, which is more robust and less error prone compared to medical imaging systems having such additional means for tracking the distance between the target of interest and the radiation source and/or the position of the target of interest and the radiation source.

A distance may be a distance from the radiation source to a surface of the target of interest or may be a distance from the radiation source to a distinct point or plane within the target of interest. A position of the target of interest may be a particular orientation of the target of interest in space. The respective distance and/or orientation may be known or derivable by the dimension and arrangement of parts of the medical imaging system.

The present invention further relates to a computer program product comprising computer-readable instructions which, when executed by a computer, cause the computer to carry out and/or control any of the methods of the present invention.

The features of the system and the method according to the present invention may be implemented by respective suitable digital or computational means, which can include, for instance, one or more computers, apps and/or networks.

The method may be at least partly computer-implemented, and may be implemented in software or in hardware, or in software and hardware. Further, the method may be carried out by computer program instructions running on data processing means that provide data processing functions.

The data processing means may be a suitable computing means, such as an electronic control module etc., which may also be a distributed computer system. The data processing means or the computer, respectively, may comprise one or more of a processor, a memory, a data interface, or the like.

The present invention further relates to a computer readable medium having stored thereon computer-readable instructions which, when executed by a computer, cause the computer to carry out and/or control any of the methods of the present invention.

The features and advantages outlined above in the context of the system and the method similarly apply to the computer program product and the computer-readable medium described herein. Likewise, any features and advantages noted with regard to the method of the present invention apply accordingly to the system of the present invention and vice versa.

A computer program (product) may be stored/distributed on a suitable medium such as an optical storage medium or a solid-state medium, supplied together with or as part of other hardware, but may also be distributed in other forms, such as via the Internet or other wired or wireless telecommunication systems.

Further features, examples, and advantages will become apparent from the following detailed description of preferred embodiments and the accompanying figures.

### BRIEF DESCRIPTION OF THE DRAWINGS

For a better understanding of the present invention, and to illustrate its practicality, figures are provided in the following and reference is made thereto. It should be understood that the figures represent only exemplary embodiments and thus in no way limit the scope of the claimed invention. Identical or like-acting elements are indicated throughout by the same reference signs. Any reference signs in the claims should not be construed as limiting the scope of the claims.

In the accompanying drawings:
Fig. 1 illustrates a schematic, not to scale view of a medical imaging system according to the present invention; and
Fig. 2 is a flowchart schematically illustrating a method for acquiring images by a medical imaging system according to the present invention.

### DETAILED DESCRIPTION OF EMBODIMENTS

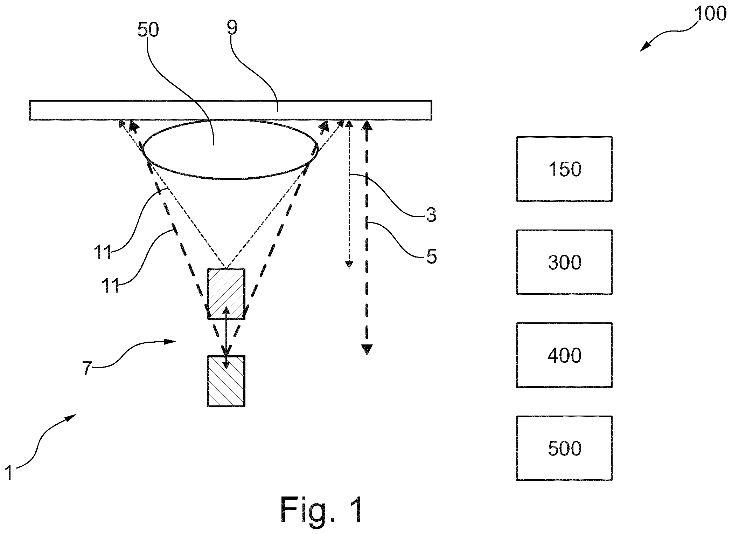

Fig. 1 illustrates an embodiment of a medical imaging system 100 according to the present invention.

The medical imaging system 100 comprises an imaging device 1 comprising a radiation source 7 and a radiation detector 9. The radiation source 7 is configured to emit a beam of radiation 11 towards a target of interest 50. The radiation passes at least partially through the target of interest 50. By passing through the target of interest 50, at least part of the radiation 11 is absorbed by the target of interest 50. The radiation detector 9 is accordingly configured to detect radiation passing by and/or through the target of interest 50.

As is indicated in the figure by the double arrow, the radiation source 7 can be positioned at different positions orthogonally to a main surface of the radiation detector 9 upon which the target of interest 50 is arranged, namely a position closer to the radiation detector 9 and the position further away from the radiation detector 9. Consequently, a first SID 3 is defined between the radiation source 7 and the detector 9 when the radiation source 7 is arranged in a first position. And a second SID 5 is defined between the radiation source 7 and the radiation detector 9 when the radiation source 7 is arranged in the second position. In the depicted embodiment, the first and second positions are not shifted laterally, such that a tomographic angle is 0.

It is clear for a skilled person that the radiation source must not necessarily be arranged such that an irradiation occurs along the vertical irradiation axis, as depicted, but can also be arranged such that the target of interest 50 is irradiated in an oblique angle with respect to the detector and/or the target of interest. In this case, the first and second SIDs are obtained by shifting the radiation source 7 accordingly along the oblique irradiation axis.

The imaging device 1 is configured to acquire a first two-dimensional image of the target of interest 50 at the first SID 3. Further, the imaging device 1 is configured to acquire a second two-dimensional image of the target of interest 50 at the second SID 5.

As will be appreciated, the radiation source 7 may also assume further and different positions with respect to the detector 9, thus potentially defining further and different SID's. At each position the medical imaging system 100 may acquire a corresponding image.

Furthermore, in the depicted embodiment, the first SID 3 is smaller than the second SID 5. However, the present invention is not limited to this particular configuration and includes, for instance, also configurations, in which the first SID 3 is larger than the second SID 5.

In the depicted embodiment, the radiation source 7 is moved only orthogonally with respect to the radiation detector 9. Thus, no lateral movement of the radiation source 7 is performed between the acquisition of the first and second two-dimensional images and the tomographic angle is essentially zero. However, in further and non-depicted embodiments, the radiation source 7 may also be moved at least partially parallel to the radiation detector 9, accordingly allowing the corresponding lateral displacement of the radiation source 7, such that the tomographic angle is greater than zero. As will be appreciated, also combined orthogonal and lateral movements of the radiation source 7 may be foreseen.

The broken arrows indicate the radiation beams 11 emitted from the radiation source 7 at the respective different positions. As is indicated, the two radiation beams 11 define a different cone shape emitted towards the radiation detector 9.

The medical imaging system 100 further comprises reconstruction means 150 configured to reconstruct at least partially a three-dimensional image based on the acquired two-dimensional images. The reconstruction means 150 accordingly employs a corresponding reconstruction algorithm.

In the illustrated embodiment, the medical imaging system 100 comprises a computer 400. In further embodiments, the computer 400 is not integrated in the medical imaging system 100.

Further, the medical imaging system 100 comprises a computer program product 300. In further embodiments, the computer program product 300 is not integrated in the medical imaging system 100.

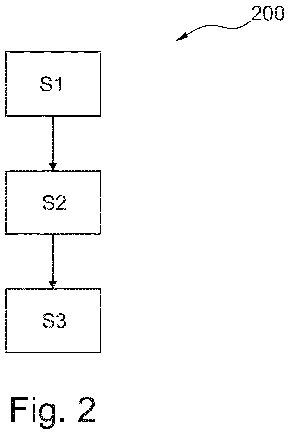

The computer program product 300 comprises computer-readable instructions which, when executed by the computer 400, cause the computer 400 to carry out and/or control a method for acquiring images by a medical imaging system 100, as described herein, for instance the method 200 described with respect to fig. 2.

In the illustrated embodiment, the medical imaging system 100 comprises a computer readable medium 500. In further embodiments, the computer readable medium 500 is not integrated in the medical imaging system 100.

The computer readable medium 500 has stored thereon computer-readable instructions which, when executed by the computer 400, cause the computer 400 to carry out and/or control a method for acquiring images by a medical imaging system 100, as described herein, for instance the method 200 described with respect to fig. 2.

Fig. 2 illustrates the flowchart of an embodiment of the method 200 for acquiring images by a medical imaging system 100, as described herein, for instance with respect to fig. 1. In the depicted embodiment, the method 200 comprises a first step S1 of acquiring, by at least one imaging device 1, at least one first two-dimensional image of a target of interest 50 at an at least one first SID 3.

Further, the method 200 comprises a second step S2 of acquiring, by the imaging device 1, at least one second two-dimensional image of the target of interest 50 at an at least one second SID 5 different to the first SID 3.

Further, the method 200 comprises a third step S3 of reconstructing, at least partially, by a reconstruction means 150, at least one three-dimensional image based on the first and second two-dimensional images.

Even though the depicted embodiment discloses two image acquisition steps S1 and S2, further configurations of the present invention may also employ further image acquisition steps at different or partially same SIDs for the first and second image acquisition steps S1 and S2.

Likewise, even though the depicted embodiment discloses one image reconstruction step S3, further configurations of the present invention may employ further image reconstruction steps, which may be based on one or more of the two-dimensional images previously acquired.

### LIST OF REFERENCE SIGNS

- 1: Imaging device
- 3: First SID
- 5: Second SID
- 7: Radiation source
- 9: Radiation detector
- 11: Radiation
- 50: Target of interest
- 100: Medical imaging system
- 150: Reconstruction means
- 200: Imaging method
- 300: Computer program product
- 400: Computer
- 500: Computer readable medium
- S1: First image acquisition step
- S2: Second image acquisition step
- S3: Reconstruction step

## Claims

1. A method (200) for acquiring images by a medical imaging system (100), the method (200) comprising the steps of:
acquiring (S1), by at least one imaging device (1), at least one first two-dimensional image of a target of interest (50) at an at least one first source image distance, SID (3),
acquiring (S2), by the imaging device (1), at least one second two-dimensional image of the target of interest (50) at an at least one second SID (5) different to the first SID (3), and
reconstructing (S3), at least partially, by a reconstruction means (150), at least one three-dimensional image based on the first and second two-dimensional images.

2. The method (200) according to the preceding claim,
wherein a difference from the first SID (3) and the second SID (5) is in a range of 50 mm to 500 mm.

3. The method (200) according to one of the preceding claims,
wherein a single first two-dimensional image at the first SID (3) and a single second two-dimensional image at the second SID (5) is acquired, and
wherein the three-dimensional image is reconstructed based on the single first and second two-dimensional images.

4. The method (200) according to one of the preceding claims,
wherein the first two-dimensional image is acquired at a first position of a radiation source (7), and
wherein the second two-dimensional image is acquired at a second position of the radiation source (7),
wherein a tomographic angle defined between the first position and the second position is essentially zero.

5. The method (200) according to one of the preceding claims 1 to 3,
wherein the first two-dimensional image is acquired at a first position of a radiation source (7), and
wherein the second two-dimensional image is acquired at a second position of the radiation source (7),
wherein a tomographic angle defined between the first position and the second position is greater than zero.

6. The method (200) according to one of the preceding claims,
wherein the first two-dimensional image is acquired at a first acquisition time,
wherein the second two-dimensional image is acquired at a second acquisition time, and
wherein the three-dimensional image is reconstructed based on the images and acquisition times of the first and second two-dimensional images.

7. A medical imaging system (100) comprising:
an imaging device (1) comprising at least one radiation source (7) configured to emit radiation (11) towards a target of interest (50) and at least one radiation detector (9) configured to detect the emitted radiation (11),
wherein the medical imaging system (100) is configured to acquire at least one first two-dimensional image of the target of interest (50) at an at least one first source image distance, SID (3), and
at least one second two-dimensional image of the target of interest (50) at an at least one second SID (5) different to the first SID (3), and
a reconstruction means (150) configured to reconstruct at least partially at least one three-dimensional image based on the first and second two-dimensional images.

8. The medical imaging system (100) according to the preceding claim,
wherein the imaging device (1) is an x-ray imaging device comprising the radiation source (7), which is an x-ray radiation source, and wherein the x-ray radiation source is configured to generate and emit x-ray radiation (11).

9. The medical imaging system (100) according to the preceding claim,
wherein the x-ray imaging device comprises an x-ray tube head,
wherein the x-ray radiation source is arranged within the x-ray tube head, and
wherein a variation between the first SID (3) and the second SID (5) is achieved by moving the x-ray tube head, and/or
wherein a variation between the first SID (3) and the second SID (5) is additionally or alternatively achieved by moving the x-ray radiation source within x-ray tube head.

10. The medical imaging system (100) according to the preceding claim,
wherein the variation between the first SID (3) and the second SID (5) is additionally or alternatively achieved by varying an emitted x-ray cone such that different SIDs are simulated.

11. The medical imaging system (100) according to one of the preceding claims,
wherein the reconstruction means (150) comprises at least one reconstruction algorithm adapted to reconstruct at least partially the at least one three-dimensional image based on the two-dimensional images,
wherein the reconstruction algorithm is based on one of the following:
training a deep-learning algorithm with computer tomography image data;
generalizing methods for three-dimensional rendering of discrete views to the domain of x-ray absorption images;
structure-from-motion techniques.

12. The medical imaging system (100) according to preceding claim,
wherein the reconstruction algorithm is additionally or alternatively designed to simulate an x-ray image with infinite SID based on the image data and the SID data.

13. The medical imaging system (100) according to one of the preceding claims,
wherein no additional means are provided for tracking a distance between the target of interest (50) and the radiation source (7) and/or a position of the target of interest (50) and the radiation source (7).

14. A computer program product (300) comprising computer-readable instructions which, when executed by a computer (400), cause the computer (400) to carry out and/or control the method (200) of any of claims 1 to 6.

15. A computer readable medium (500) having stored thereon computer-readable instructions which, when executed by a computer (400), cause the computer (400) to carry out and/or control the method (200) of any of claims 1 to 6.
